# EUROPEAN PATENT SPECIFICATION

(11) **EP 1 603 573 B1**
(45) Date of publication and mention of the grant of the patent: **21.05.2008**
(21) Application number: 04709596.3
(22) Date of filing: 10.02.2004
(51) Int. Cl.: A61K 31/60, A61K 31/519, A61K 31/4184, A61P 9/00

(54) **USE OF DIPYRIDAMOLE IN COMBINATION WITH ACETYLSALICYLIC ACID AND AN ANGIOTENSIN II ANTAGONIST FOR STROKE PREVENTION**
KOMBINATIONSPRÄPARATE ZUR VERHINDERUNG VON SCHLAGANFÄLLEN, WELCHE DIPYRIDAMOLE, ACETYLSALICYLSÄURE UND EINEN ATII ANTAGONISTEN ENTHALTEN
UTILISATION DE DIPYRIDAMOLE EN ASSOCIATION AVEC DE L'ACIDE ACETYLSALICYLIQUE ET UN ANTAGONISTE DE L'ANGIOTENSINE II AUX FINS DE LA PREVENTION DES ACCIDENTS CEREBRO-VASCULAIRES

(30) Priority: 13.02.2003 DE 10306179; 08.08.2003 EP 03018212
(43) Date of publication of application: 14.12.2005
(60) Divisional application: 06121998.6
(73) Proprietor: Boehringer Ingelheim International GmbH, 55216 Ingelheim am Rhein (DE); Boehringer Ingelheim Pharma GmbH & Co.KG, 55216 Ingelheim am Rhein (DE)
(72) Inventor: HILBRICH, Lutz, 55252 Mainz-Kastel (DE); GILBERT, James C., Bethlehem, Connecticut 06751 (US); HUMPHREYS,David, Michael, I-52031 Anghiari (IT); RIEDEL, Axel, Maselheim D-88437 (DE)
(86) International application number: PCT/EP2004/001208
(87) International publication number: WO 2004/071385

(56) References cited:
- WO-A-01/15673
- WO-A-01/30353
- "AGGRENOX: A COMBINATION OF ANTIPLATELET DRUGS FOR STROKE PREVENTION" MEDICAL LETTER ON DRUGS AND THERAPEUTICS, NEW ROCHELLE, NY, US, vol. 42, no. 1071, 7 February 2000 (2000-02-07), pages 11-12, XP000933411 ISSN: 0025-732X
- SCHEEN A J: "LA COMBINAISON FIXE DIPYRIDAMOLE - ACIDE ACETYLSALICYLIQUE (AGGRENOX) EXTENDED-RELEASE DIPYRIDAMOLE/ASPIRIN (AGGRENOXÄRHOÜ" REVUE MEDICALE DE LIEGE, LIEGE, BE, vol. 55, no. 10, 2000, pages 957-959, XP009033957 ISSN: 0370-629X
- MALININ A I ET AL: "AGGRENOX (EXTENDED-RELEASE DIPYRIDAMOLE AND LOW-DOSE ASPIRIN IN COMBINATION): PROTECTING PLATELETS FROM EXCESSIVE ACTIVATION IN PATIENTS WITH VASCULAR EVENTS" HEART DRUG, KARGER, BASEL, CH, vol. 2, no. 2, March 2002 (2002-03), pages 93-104, XP009033969 ISSN: 1422-9528
- BOEHRINGER INGELHEIM NEWS, [Online] XP002297102 13-02-2003 Retrieved from the Internet: URL:http://www.boehringer-ingelheim.com/co rporate/asp/archive/adetail.asp?ID=594> [retrieved on 2004-09-20]

## Description

### Field of the Invention

This invention relates to a method of preventing stroke or reducing the risk of stroke in a patient in need thereof, especially in a patient at risk for a stroke or a secondary stroke, using dipyridamole in combination with acetylsalicylic acid (ASA) and an angiotensin II antagonist selected from the group consisting of candesartan, eprosartan, irbesartan, losartan, telmisartan, valsartan, olmesartan and tasosartan, a pharmaceutical composition comprising a combination of dipyridamole, acetyl salicylic acid and an angiotensin II antagonist, and the use of dipyridamole for the manufacture of a corresponding pharmaceutical composition comprising a combination of dipyridamole, acetyl salicylic acid and an angiotensin II antagonist.

### Background of the Invention

Dipyridamole {2,6-bis(diethanolamino)-4,8-dipiperidino-pyrimido[5,4-d]pyrimidine}, closely related substituted pyrimido-pyrimidines and their preparation have been described in e.g. U.S.Patent 3,031,450. Dipyridamole was introduced as a *coronary vasodilator* in the early 1960s. It is also well known having *platelet aggregation inhibitor properties* due to the inhibition of adenosine uptake. Subsequently, dipyridamole was shown to reduce thrombus formation in a study of arterial circulation of the brain in a rabbit model. These investigations led to its use as an *antithrombotic agent,* it soon became the therapy of choice for such applications as stroke prevention, maintaining the patency of coronary bypass and valve-replacement, as well as for treatment prior to coronary angioplasty.

Furthermore, the European Stroke Prevention Study 2 (ESPS-2; J Neurol Sci. 1996; 143: 1-13; Neurology 1998; 51: 17-19) proved that treatment by dipyridamole alone was as effective as low-dose aspirin in the reduction of stroke risk, and combination therapy with dipyridamole and aspirin was more than twice as effective as aspirin alone.

Dipyridamole appears to inhibit thrombosis through multiple mechanisms. Early studies showed that it inhibits the uptake of adenosine, which was found to be a potent endogenous anti-thrombotic compound. Dipyridamole was also shown to inhibit cyclic AMP phosphodiesterase, thereby increasing intracellular c-AMP.

Dipyridamole also has *antioxidant properties* (Free Radic. Biol. Med. 1995; 18: 239-247) that may contribute to its antithrombotic effect. When oxidized, low density lipoproteins become recognized by the scavenger receptor on macrophages, which is assumed to be the necessary step in the development of atherosclerosis (Ann. Rev. Med. 1992; 43: 219-25).

The inhibition of free radical formation by dipyridamole has been found to inhibit fibrinogenesis in experimental liver fibrosis (Hepatology 1996; 24: 855-864) and to suppress oxygen radicals and proteinuria in experimental animals with amino-nucleoside nephropathy (Eur. J. Clin. Invest. 1998; 28: 877-883; Renal Physiol. 1984; 7: 218-226). Inhibition of lipid peroxidation also has been observed in human nonneoplastic lung tissue (Gen. Pharmacol. 1996; 27: 855-859).

Angiotensin (ANG) II plays a major role in pathophysiology, especially as the most potent blood pressure increasing agent in humans. ANG II antagonists therefore are suitable for treating elevated blood pressure and congestive heart failure in a mammal. Examples of ANG II antagonists are described in EP-A-0 502 314, EP-A-0 253 310 , EP-A-0 323 841, EP-A-0 324 377, US-A-4,355,040 and US-A-4,880,804. Specific ANG II antagonists are sartans such as candesartan, eprosartan, irbesartan, losartan, telmisartan or valsartan, furthermore olmesartan and tasosartan.

It is known that ANG II, besides its blood pressure increasing effect, additionally features growth promoting effects contributing to left ventricular hypertrophy, vascular thickening, atherosclerosis, renal failure and stroke. Bradykinin, on the other hand, exerts vasodilating and tissue protective actions, as disclosed for instance by W.

Wienen et al.: Antihypertensive and renoprotective effects of telmisartan after long term treatment in hypertensive diabetic (D) rats, 2nd. Int. Symposium on Angiotensin II Antagonism, February 15-18, 1999, The Queen Elizabeth II Conference Center, London, UK, Book of Abstracts, Abstract No. 50.

Losartan and irbesartan provide a renoprotective effect found within first clinical trials, as disclosed for instance by S. Andersen et al.: Renoprotective effects of angiotensin II receptor blockade in type 1 diabetic patients with diabetic nephropathy, Kidney Int 57 (2), 601-606 (2000).

ANG II antagonists selectively block the AT₁ receptor, leaving the AT₂ receptor, which plays a role in anti-growth and tissue regenerative actions, unopposed. Completed clinical trials with Ang II antagonists appear to display similar blood pressure reducing and tissue protective effects as with ACE inhibitors, as disclosed forb instance by D.H.G. Smith et al.: Once-daily telmisartan compared with enalapril in the treatment of hypertension, Adv. Ther 1998, 15: 229-240, and by B.E. Karlberg et al.: Efficacy and safety of telmisartan, a selective AT1 receptor antagonist, compared with enalapril in elderly patients with primary hypertension, J Hypertens 1999, 17: 293-302.

EP-A-1 013 273 discloses the use of AT₁ receptor antagonists or AT₂ receptor modulators for treating diseases associated with an increase of AT₁ receptors in subepithelial area or increase of AT₂ receptors in the epithelia, especially for treatment of several lung diseases.

### Summary of the Invention

It has now surprisingly been found that dipyridamole when administered in combination with acetylsalicylic acid and an angiotensin II antagonist selected from the group consisting of candesartan, eprosartan, irbesartan, losartan, telmisartan, valsartan, olmesartan and tasosartan provides a stroke preventing effect superior to conventional medications or treatment regimes, for instance a combination regime of clopidogrel together with acetyl salicylic acid, especially in a patient at risk for a stroke or a secondary stroke.

ASA inhibits aggregation through direct effects on the platelet, in more detail, by irreversibly acetylating platelet cyclooxygenase, thus inhibiting the production of thromboxane, which is strongly thrombotic. In high doses, however, aspirin crosses over into endothelial cells (N. Eng. J. Med. 1984; 311: 1206-1211), where it interrupts the production of prostacyclin, a potent natural inhibitor of platelet aggregation and by-product of the "arachidonic cascade" (N. Engl. J. Med. 1979; 300: 1142-1147). These observations led to the concept of low-dose antiplatelet therapy with ASA to maximize inhibition of thromboxane while minimizing the loss of prostacyclin (Lancet 1981; 1: 969-971). In the method of prevention according to the invention a combination of low-dose ASA with dipyridamole and an angiotensin II antagonist selected from the group consisting of candesartan, eprosartan, irbesartan, losartan, telmisartan, valsartan, olmesartan and tasosartan is preferred.

Viewed from one aspect the present invention provides a method of stroke prevention or reducing the risk of stroke or secondary stroke in a patient in need thereof, especially in a patient with elevated risk for stroke, e.g. in hypertensive patients or patients suffering from cerebrovascular disorders, said method comprising administering to said patient an effective amount of a pharmaceutical composition comprising dipyridamole or a pharmaceutically acceptable salt thereof in combination with ASA and an angiotensin II antagonist. The main risk for a second stroke is a prior stroke due to degenerative processes in the wall of blood vessels supplying the brain. Patients at high risk of a second stroke with all its consequences readily seek preventive treatment. The vascular pathobiology of ischaemic stroke is multiple and antithrombotic mechanisms in the cerebro-vascular microenvironment beyond platelet inhibition seem to be potentially disease-modifying and a means of reducing ischaemic stroke.

Viewed from a second aspect the present invention provides a pharmaceutical composition comprising dipyridamole or a pharmaceutically acceptable salt thereof in combination with ASA and an angiotensin II antagonist, adapted for simultaneous, separate or sequential administration.

The pharmaceutical composition according to the invention is meant to comprise a fixed dose combination comprising the active ingredients in one formulation together as well as
a kit of parts comprising
(a) a first containment containing a pharmaceutical composition comprising a therapeutically effective of dipyridamole; and
(b) a second containment containing a pharmaceutical composition comprising acetylsalicylic acid and a pharmaceutically acceptable carrier; and
(c) a third containment containing a pharmaceutical composition comprising an angiotensin II antagonist selected from the group consisting of candesartan, eprosartan, irbesartan, losartan, telmisartan, valsartan, olmesartan and tasosartan.

Viewed from a different aspect the present invention provides the use of dipyridamole or a pharmaceutically acceptable salt thereof in combination with ASA and an angiotensin II antagonist for the manufacture of a pharmaceutical composition for stroke prevention or reducing the risk of stroke or secondary stroke in a patient in need thereof.

### Detailed Description of the Invention

The invention provides a new and improved approach for stroke prevention or reducing the risk of stroke or secondary stroke in a patient in need thereof, especially in a patient with elevated risk for stroke; comprising administering to the patient an effective amount of a pharmaceutical composition containing as acive ingredients dipyridamole or a pharmaceutically acceptable salt thereof in combination with ASA and an angiotensin II antagonist selected from the group consisting of candesartan, eprosartan, irbesartan, losartan, telmisartan, valsartan, olmesartan and tasosartan.

In the method of the invention any of the oral dipyridamole retard, instant or the parenteral formulations on the market may be used, the retard formulations being preferred, for instance those available under the trademark Persantin^{®}, or, already in combination with ASA the formulations available under the trademark Asasantin^{®} or Aggrenox^{®}. Suitable dipyridamole retard formulations are disclosed in EP-A-0032562, instant formulations are disclosed in EP-A-0068191 and combinations of ASA with dipyridamole are disclosed in EP-A-0257344. The Angiotensin II antagonist that may be used in the method of prevention of the invention are candesartan, eprosartan, irbesartan, losartan, telmisartan (trademark Micardis^{®}), valsartan, olmesartan or tasosartan, including the salts thereof or polymorphs thereof, using for instance the dosages disclosed in Rote Liste^{®}2003, Editio Cantor Verlag Aulendorf, Germany, or in Physician's Desk Reference, 57 edition, 2003.

In the method of prevention according to the invention a plasma level of dipyridamole of about 0.2 to 5 µmol/L, preferably of about 0.5 to 2 µmol/L or particularly of about 0.8 to 1.5 µmol/L may be maintained. Dipyridamole can be administered orally in a daily dosage of 50 to 750 mg, preferably 100 to 500 mg, most preferred 200 to 450 mg, for instance 200 mg twice a day.

With respect to ASA this component of the ternary medication may be administered orally in a daily dosage of 10 to 200 mg, preferably 25 to 100 mg, most preferred 30 to 75 mg, for instance 25 mg twice a day.

With respect to the third component the angiotensin II antagonist telmisartan is preferred. This component can be administered orally in a daily dosage of 10 to 200 mg, for instance in a daily dosage of 20, 40, 80 or 160 mg, preferably in a daily dosage of 40 to 160 mg, most preferred 60 to 100 mg, for instance 20 or 40 mg once a day.

A specific method of prevention according to the invention comprises the combination of dipyridamole administered orally 200 mg twice a day, ASA administered orally 25 mg twice a day and telmisartan administered orally 20, 40 or 80 mg once a day.

With respect to all aspects of the invention the combination of dipyridamole, ASA and telmisartan is preferred, especially in the oral dosages indicated hereinbefore as most preferred.

## Claims

1. A pharmaceutical composition comprising dipyridamole or a pharmaceutically acceptable salt thereof, acetyl salicylic acid (ASA) and an angiotensin II antagonist selected from the group consisting of
candesartan, eprosartan, irbesartan, losartan, telmisartan, valsartan, olmesartan and tasosartan, including the salts thereof or polymorphs thereof,
as a combined preparation for simultaneous, separate or sequential use.

2. The pharmaceutical composition according to claim 1 as a fixed dose combination.

3. The pharmaceutical composition according to claim 1 as a kit of parts comprising
(a) a first containment containing a pharmaceutical composition comprising a therapeutically effective of dipyridamole; and
(b) a second containment containing a pharmaceutical composition comprising ASA and a pharmaceutically acceptable carrier; and
(c) a third containment containing a pharmaceutical composition comprising an angiotensin II antagonist selected from the group consisting of candesartan, eprosartan, irbesartan, losartan, telmisartan, valsartan, olmesartan and tasosartan, including the salts thereof or polymorphs thereof.

4. The pharmaceutical composition according to one of claims 1 to 3 for oral administration.

5. The pharmaceutical composition according to one of claims 1 to 4 wherein the angiotensin II antagonist is telmisartan.

6. The use of dipyridamole or a pharmaceutically acceptable salt thereof in combination with acetylsalicylic acid (ASA) and an angiotensin II antagonist selected from the group consisting of
candesartan, eprosartan, irbesartan, losartan, telmisartan, valsartan, olmesartan and tasosartan, including the salts thereof or polymorphs thereof,
for the manufacture of a pharmaceutical composition for stroke prevention or reducing the risk of stroke or secondary stroke in a patient.

7. The use of claim 6, wherein the pharmaceutical composition is a fixed dose combination.

8. The use of claim 6, wherein the pharmaceutical composition is a kit of parts comprising
(a) a first containment containing a pharmaceutical composition comprising a therapeutically effective of dipyridamole; and
(b) a second containment containing a pharmaceutical composition comprising ASA and a pharmaceutically acceptable carrier; and
(c) a third containment containing a pharmaceutical composition comprising an angiotensin II antagonist selected from the group consisting of candesartan, eprosartan, irbesartan, losartan, telmisartan, valsartan, olmesartan and tasosartan, including the salts thereof or polymorphs thereof.

9. The use of one of claims 6 to 8, wherein the pharmaceutical composition is for oral administration.

10. The use of one of claims 6 to 9, wherein the angiotensin II antagonist is telmisartan.

## Patentansprüche

1. Pharmazeutische Zusammensetzung, umfassend Dipyridamol oder ein pharmazeutisch akzeptables Salz hiervon, Acetylsalicylsäure (ASS) und einen Angiotensin-II-Antagonisten, ausgewählt aus der Gruppe, bestehend aus
Candesartan, Eprosartan, Irbesartan, Losartan, Telmisartan, Valsartan, Olmesartan und Tasosartan, einschließlich der Salze oder Polymorphe hiervon,
als eine kombinierte Zubereitung zur gleichzeitigen, getrennten oder aufeinanderfolgenden Verwendung.

2. Pharmazeutische Zusammensetzung nach Anspruch 1 als Kombination mit fester Dosierung.

3. Pharmazeutische Zusammensetzung nach Anspruch 1 als Kit von Teilen, umfassend
(a) ein erstes Behältnis, enthaltend eine pharmazeutische Zusammensetzung, umfassend therapeutisch wirksames Dipyridamol; und
(b) ein zweites Behältnis, enthaltend eine pharmazeutische Zusammensetzung, umfassend ASS und einen pharmazeutisch akzeptablen Träger; und
(c) ein drittes Behältnis, enthaltend eine pharmazeutische Zusammensetzung, umfassend einen Angiotensin-II-Antagonisten, ausgewählt aus der Gruppe, bestehend aus Candesartan, Eprosartan, Irbesartan, Losartan, Telmisartan, Valsartan, Olmesartan und Tasosartan, einschließlich der Salze oder Polymorphe hiervon.

4. Pharmazeutische Zusammensetzung nach einem der Ansprüche 1 bis 3 zur oralen Verabreichung.

5. Pharmazeutische Zusammensetzung nach einem der Ansprüche 1 bis 4, worin der Angiotensin-II-Antagonist Telmisartan darstellt.

6. Verwendung von Dipyridamol oder einem pharmazeutisch akzeptablen Salz hiervon in Kombination mit Acetylsalicylsäure (ASS) und einem Angiotensin-II-Antagonisten, ausgewählt aus der Gruppe, bestehend aus
Candesartan, Eprosartan, Irbesartan, Losartan, Telmisartan, Valsartan, Olmesartan und Tasosartan, einschließlich der Salze oder Polymorphe hiervon,
zur Herstellung einer pharmazeutischen Zusammensetzung zur Schlaganfallvorbeugung oder Reduzierung des Risikos eines Schlaganfalls oder eines zweiten Schlaganfalls bei einem Patienten.

7. Verwendung nach Anspruch 6, wobei die pharmazeutische Zusammensetzung eine Kombination mit fester Dosierung ist.

8. Verwendung nach Anspruch 6, worin die pharmazeutische Zusammensetzung ein Kit von Teilen darstellt, umfassend
(a) ein erstes Behältnis, enthaltend eine pharmazeutische Zusammensetzung, umfassend therapeutisch wirksames Dipyridamol; und
(b) ein zweites Behältnis, enthaltend eine pharmazeutische Zusammensetzung, umfassend ASS und einen pharmazeutisch akzeptablen Träger; und
(c) ein drittes Behältnis, enthaltend eine pharmazeutische Zusammensetzung, umfassend einen Angiotensin-II-Antagonisten, ausgewählt aus der Gruppe, bestehend aus Candesartan, Eprosartan, Irbesartan, Losartan, Telmisartan, Valsartan, Olmesartan und Tasosartan, einschließlich der Salze oder Polymorphe hiervon.

9. Verwendung nach einem der Ansprüche 6 bis 8, worin die pharmazeutische Zusammensetzung zur oralen Verabreichung ist.

10. Verwendung nach einem der Ansprüche 6 bis 9, worin der Angiotensin-II-Antagonist Telmisartan darstellt.

## Revendications

1. Composition pharmaceutique comprenant du dipyridamole ou un sel pharmaceutiquement acceptable de celui-ci, de l'acide acétylsalicylique (ASA) et un antagoniste d'angiotensine II choisi dans le groupe consistant en
candesartan, eprosartan, irbesartan, losartan, telmisartan, valsartan, olmesartan et tasosartan, y compris leurs sels ou leurs polymorphes,
sous forme d'une préparation combinée pour l'utilisation simultanée, séparée ou successive.

2. Composition pharmaceutique selon la revendication 1 sous forme d'une combinaison à dose fixée.

3. Composition pharmaceutique selon la revendication 1 sous forme d'un kit de parties comprenant
(a) un premier récipient contenant une composition pharmaceutique comprenant une thérapeutiquement efficace de dipyridamole ; et
(b) un second récipient contenant une composition pharmaceutique comprenant ASA et un vecteur pharmaceutiquement acceptable ; et
(c) un troisième récipient contenant une composition pharmaceutique comprenant un antagoniste d'angiotensine II choisi dans le groupe consistant en candesartan, eprosartan, irbesartan, losartan, telmisartan, valsartan, olmesartan et tasosartan, y compris leurs sels ou leurs polymorphes.

4. Composition pharmaceutique selon l'une des revendications 1 à 3 pour l'administration orale.

5. Composition pharmaceutique selon l'une des revendications 1 à 4 où l'antagoniste d'angiotensine II est le telmisartan.

6. Utilisation du dipyridamole ou d'un sel pharmaceutiquement acceptable de celui-ci en combinaison avec l'acide acétylsalicylique (ASA) et un antagoniste d'angiotensine II choisi dans le groupe consistant en
candesartan, eprosartan, irbesartan, losartan, telmisartan, valsartan, olmesartan et tasosartan, y compris leurs sels ou leurs polymorphes,
pour la fabrication d'une composition pharmaceutique pour la prévention des attaques ou la réduction du risque d'attaque ou d'attaque secondaire chez un patient.

7. Utilisation selon la revendication 6 où la composition pharmaceutique est une combinaison à dose fixée.

8. Utilisation selon la revendication 6 où la composition pharmaceutique est un kit de parties comprenant
(a) un premier récipient contenant une composition pharmaceutique comprenant une thérapeutiquement efficace de dipyridamole ; et
(b) un second récipient contenant une composition pharmaceutique comprenant ASA et un vecteur pharmaceutiquement acceptable ; et
(c) un troisième récipient contenant une composition pharmaceutique comprenant un antagoniste d'angiotensine II choisi dans le groupe consistant en
candesartan, eprosartan, irbesartan, losartan, telmisartan, valsartan, olmesartan et tasosartan, y compris leurs sels ou leurs polymorphes.

9. Utilisation selon l'une des revendications 6 à 8 où la composition pharmaceutique est destinée à l'administration orale.

10. Utilisation selon l'une des revendications 6 à 9 où l'antagoniste d'angiotensine II est le telmisartan.
